# EUROPEAN PATENT APPLICATION

(11) **EP 3 437 620 A1**
(43) Date of publication of application: **06.02.2019**
(21) Application number: 17774071.9
(22) Date of filing: 06.03.2017
(51) Int. Cl.: A61J 1/10, A61J 1/05, B65D 65/02, C08L 23/08, C08L 23/20, C08L 53/02, A61K 35/14, A61K 35/16

(54) **COMPOSITION FOR PLATELET STORAGE CONTAINER, SHEET FOR PLATELET STORAGE CONTAINER, AND PLATELET STORAGE CONTAINER**

(30) Priority: 28.03.2016 JP 2016064017
(71) Applicant: Kawasumi Laboratories, Inc., Saiki-shi, Oita 876-0121 (JP)
(72) Inventor: MORISHIMA, Asa, Bungo-Ono-shi Oita 879-7153 (JP); ITO, Takatoshi, Bungo-Ono-shi Oita 879-7153 (JP); MORITA, Norifumi, Bungo-Ono-shi Oita 879-7153 (JP)
(74) Representative: Dennemeyer & Associates S.A.
(86) International application number: PCT/JP2017/008668
(87) International publication number: WO 2017/169526

(57) **Abstract**

A composition for a platelet storage container that enables continuous and stable long-term storage of high number of platelets has a composition of: (a) 30-40% by weight of a hydrogenated block copolymer comprising a polymer block A composed mainly of a vinyl aromatic compound and a polymer block B composed mainly of a conjugated diene; (b) 35-65% by weight of polyethene having a density of 0.860-0.900 g/cm3, the polyethylene being obtained by copolymerizing ethylene and an α-olefin; and (c) 5-25% by weight of poly(methylpentene), or of a (co)polymer composed mainly of methylpentene with ethylene or an α-olefin, component (c) having an MFR of 3-20 g/10 min, a density of 800-900 g/cm3, and a melting point (Tm) of 180°C or lower as measured by differential scanning calorimetry (DSC).

## Description

### Technical Field

The present invention relates to: a composition for a platelet storage container consisting of a thermoplastic elastomer composition, where the composition is superior in flexibility, transparency, thermal resistance, strength, and molding and/or forming properties, and enables formation of a container particularly superior in gas permeability; and a sheet and a platelet storage container formed from the composition.

### Background Art

The present inventors proposed an invention relating to an elastomeric resin composition which does not cause inhibition of platelet functions due to elution of a plasticizer (e.g., DDP), which occurs for conventional containers with non-rigid vinyl chloride resin. Specifically, the present inventors disclosed, in PTL 1 (Japanese Patent Application Laid-Open No. H08-188687), a container for medical use with an elastomer composition which is superior in flexibility, transparency, thermal resistance, strength, and molding and/or forming properties and is nontoxic and odorless, where the container is superior in gas permeability, biocompatibility, and platelet storability. The container for medical use consists of a four-component elastomer composition containing the following (e), (f), (g), and (h):
(e) 30 to 85wt% of a block copolymer prepared by hydrogenation of a block copolymer including copolymer block A composed mainly of at least two vinyl aromatic compounds and polymer block B composed mainly of at least one conjugated diene;
(f) 5 to 30wt% of a propylene homopolymer or a copolymer composed mainly of propylene;
(g) 5 to 60wt% of an ethylene copolymer; and
(h) 3 to 20wt% of poly(methylpentene) or a copolymer of methylpentene and an α-olefin.

The invention described in PTL 1 was made with a focus on a close relation between platelet storability and gas permeability. The present applicants have reported in PTL 1 that the container for medical use is superior in platelet storability on the basis of results that when platelets were stored in a container for medical use in Example 6 or 10, which had high gas permeability, the decrease of pH was lower, the aggregation ability of platelets was less degraded, the deformability of platelets was less degraded, and the decrease of the percent hypotonic shock response (%HSR) was lower than for storage in a container for medical use in Comparative Example 3, which was poor in gas permeability, even in long-term storage (see paragraphs [0028], [0029], Tables 5 and 10 in PTL 1).

### Citation List

### Patent Literature

PTL 1 Japanese Patent Application Laid-Open No. H08-188687 (Claim 2 in CLAIMS)

### Summary of Invention

### Technical Problem

Platelets are stored and used as a platelet concentrate. While the maximum storage period of platelet concentrates is currently three days in Japan, it has already become five to seven days in the mainstream in the world. In contrast to the situation in the Japanese domestic market that most platelet concentrate are consist of 10 units (platelet count: 2.0 × 10¹¹ cells, volume: 200 ± 40 mL), platelets are stored in high number and large volumes (e.g., 10 to 30 units, 250 to 350 mL) in the global market. In such trends, a novel platelet storage container which enables storage of platelets in higher units and larger volume for a longer period of time is considered essential also in Japan, and such a platelet storage container is strongly required by medical personnel.

### Solution to Problem

In view of such circumstances, the present applicants diligently examined to solve the above-mentioned problem, with a focus on poly(methylpentene) or copolymers of methylpentene and an α-olefin, and as a result found that a platelet storage container formed from a polymer composition containing a specific amount of the (co)polymer having specific physical properties, with the specified amounts of other resin components, has extremely high oxygen gas (O₂) permeability. That is, it was found that significantly improved gas permeability can be provided when a platelet storage container is produced from the polymer composition, as compared with when a container is produced from the composition disclosed in PTL 1.

The present invention was completed on the basis of such finding. The present invention provides: a composition consisting of an elastomer composition for a platelet storage container, where the composition enables production of a platelet storage container having high gas permeability to allow long-term storage of platelets; and a sheet for a platelet storage container, the sheet obtained by shaping the composition. Further, the composition in the present invention has higher blendability provided through specification of the (co)polymer having specific physical properties and use of the (co)polymer with a specific quantitative ratio, and this higher blendability is expected to provide advantages to lead to reduced variation of the physical properties and good heat-sealability of sheets to be produced.

Specifically, the present invention is as follows.
[1] According to the present invention, a composition for a platelet storage container is provided, the composition comprising:
   (a) 30 to 40wt% of a block copolymer prepared by hydrogenation of a block copolymer comprising a polymer block A comprising at least two vinyl aromatic compounds and a polymer block B comprising at least one conjugated diene;
   (b) 35 to 65wt% of polyethylene obtained by copolymerizing ethylene and a C₃₋₁₂ α-olefin with a metallocene catalyst, the polyethylene having a density of 0.860 to 0.900 g/cm³ and a maximum melting peak temperature (Tp) of 90°C or lower as measured by differential scanning calorimetry (DSC); and
   (c) 5 to 25wt% of poly(methylpentene) or a copolymer comprising methylpentene with ethylene or an α-olefin,
   in which the component (c) has a Melt Flow Rate (MFR) of 3 to 20 g/10 min, a density of 800 to 900 g/cm³, and a melting point (Tm) of 180°C or lower as measured by the DSC.
[2] According to the present invention, a sheet for a platelet storage container is provided, the sheet obtained by shaping the composition according to [1] described above, in which:
   a thickness of the sheet is 150 to 400 µm, and
   an oxygen permeability per unit thickness of the sheet is 850 ml·mm/m²·day·atm or higher, a carbon dioxide permeability per unit thickness of the sheet is 4,500 ml·mm/m²·day·atm or higher, and a carbon dioxide/oxygen permeability ratio (CO₂ permeability/O₂ permeability) per unit thickness of the sheet is 6.0 or lower.
[3] According to the present invention, the sheet for a platelet storage container according to [2] described above is provided, in which:
   a 100% modulus of the sheet is 2.7 to 5 MPa, and
   a breaking strength of the sheet is 20 MPa or higher.
[4] According to the present invention, provided is a platelet storage container obtained by forming the sheet for a platelet storage container according to [2] or [3] described above into a bag.
[5] According to the present invention, provided is a platelet storage container obtained by shaping the composition according to [1] described above into a tube and then shaping the tube into a bottle, or obtained by shaping the composition into a bottle.

### Advantageous Effects of Invention

The composition for a platelet storage container according to the present invention can be shaped, for example, into a sheet for use. The shaped sheet is superior in strength (resistance to mechanical force), molding and/or forming properties, and resistance to platelet adhesion, and, in particular, significantly superior in oxygen permeability, and the carbon dioxide gas/oxygen permeability ratio (CO₂ permeability/O₂ permeability) is kept well-balanced within a preferred range in spite of the high oxygen permeability. Therefore, the shaped sheet enables continuous and stable storage of high number of platelets, and is expected to facilitate long-term storage of platelets.

### Description of Embodiments

The composition for a platelet storage container according to the present invention (hereinafter, occasionally referred to as "the composition according to the present invention", or simply to "the composition") contains a three-component resin composition consisting of a styrene-butadiene hydrogenated block copolymer as component (a), an ethylene copolymer as component (b), and a methylpentene (co)polymer as component (c).

### ((a) Hydrogenated block copolymer)

The hydrogenated block copolymer as component (a) is a block copolymer obtained by hydrogenation of a block copolymer including at least two polymer block A composed mainly of vinyl aromatic compounds such as styrene and polymer block B composed mainly of at least one conjugated diene such as butadiene, and a representative example of the hydrogenated block copolymer is a block copolymer obtained by hydrogenation of a styrene-butadiene block copolymer. For example, the hydrogenated block copolymer has a structure (repeating units) such as A-B-A, B-A-B-A, A-B-A-B-A, B-A-B-A-B, (A-B)₄-Si, (B-A-B)₄-Si, or (B-A-B)₄-Sn, where Si represents a silicon atom and Sn represents a tin atom.

A hydrogenated block copolymer having a structure (repeating units) obtained by hydrogenation of a block copolymer including polymer block B composed mainly of a conjugated diene at at least one polymer end is particularly suitable for production of a shaped product which is superior in flexibility and processability, and has a lower possibility of the occurrence of anisotropy. The hydrogenated block copolymer has, for example, a structure (repeating units) such as

B-A-B-A,

B-A-B-A-B,

(B-A-B)₄-Si,

(B-A-B)₃-Si-R,

(B-A-B)₄-Sn,

(B-A-B)₃-Sn-R,

(B-A-B)₂-Si-R,R'

(A-B),(B-A-B)-Si-R,R',

or

(A-B),(B-A-B)₂-Si-R

where R and R' each represent a C₁₋₈ alkyl group, and may be identical or different; Si represents a silicon atom; and Sn represents a tin atom.

The phrase "composed mainly of' as used herein means that the monomer unit accounts for at least 50mass% or more, preferably 70mass% or more, of the total of constitutional units included in a polymer block.

In polymer block A composed mainly of a vinyl aromatic compound such as styrene and polymer block B composed mainly of a conjugated diene such as butadiene, the distribution of the vinyl aromatic compound or conjugated diene in each polymer block may be any of random distribution, tapered distribution (distribution in which the frequency of the appearance of the monomer component increases or decrease along the direction from one end to the other end of the molecular chain), partial block distribution, and any combination thereof. In the case that two or more polymer blocks A or polymer blocks B are present in the (a) hydrogenated block copolymer, the two or more polymer blocks may consist only of polymer blocks having the same structure, or include polymer blocks having different structures.

The range of the number-average molecular weight of the hydrogenated block copolymer is preferably 50,000 to 250,000, and more preferably 70,000 to 200,000. The strength and oil resistance of a shaped product derived from the composition are insufficient if the number-average molecular weight is lower than 50,000, and the molding and/or forming properties of the composition is deteriorated if the number-average molecular weight is higher than 250,000, and thus such number-average molecular weight is not preferred.

The molecular weight distribution [the ratio of the weight-average molecular weight (Mw) to the number-average molecular weight (Mn), (Mw/Mn)] of the copolymer is preferably 10 or lower, more preferably 5 or lower, and even more preferably 2 or lower.

In Examples described later, a hydrogenated block copolymer satisfying these physical properties is used.

### ((b) Ethylene copolymer)

The ethylene copolymer used as component (b) is a copolymer of ethylene and an α-olefin (hereinafter, occasionally expressed as "polyethylene", simply), and a product obtained by polymerizing ethylene and an α-olefin with a metallocene catalyst is used for component (b). The polyethylene produced with the catalyst has a narrow molecular weight distribution, and hence the growth of the crystal nucleus tends to homogeneously proceed to give a homogeneous crystal with a thin lamellar structure. For this reason, the shaped sheet has higher gas permeability.

The density of the ethylene copolymer is 0.860 to 0.900 g/cm³, and preferably 0.870 to 0.890 g/cm³. Ethylene copolymers having a density of lower than 0.860 g/cm³ impart lower strength to the shaped sheet, and tend to generate tackiness on the surface of the shaped product to deteriorate the appearance of the shaped product, and thus such ethylene copolymers are not preferred. Ethylene copolymers having a density of higher than 0.900 g/cm³ impart lower oxygen permeability to the shaped sheet, and thus are not preferred.

The maximum melting peak temperature (Tmp) of the ethylene copolymer is 90°C or lower as measured by differential scanning calorimetry (DSC). The maximum melting peak temperature is preferably the maximum peak temperature in a thermogram to be acquired by using a differential scanning calorimeter when the temperature is raised at a rate of 4°C/min. To increase the oxygen permeability of the platelet storage container according to the present invention, the maximum melting peak temperature (Tmp) of the ethylene copolymer is preferably 90°C or lower as measured by differential scanning calorimetry (DSC).

In Examples described later, an ethylene copolymer satisfying these physical properties is used.

### ((c) Methylpentene (co)polymer)

The methylpentene (co)polymer used as component (c) in the present invention refers to both a homopolymer of methylpentene [poly(methylpentene)] and a copolymer of methylpentene and an α-olefin such as ethylene and propylene, the copolymer composed mainly of methylpentene. These homopolymer and copolymer can be used singly, or as a mixture of the homopolymer and the copolymer.

The methylpentene (co)polymer as component (c) preferably has an Melt Flow Rate (MFR) of 3 to 20 g/ 10 min and a density, p, of 800 to 900 g/cm³.

In particular, it is desired for the methylpentene (co)polymer to have a melting point (Tm) of 180°C or lower, preferably of 130°C or lower, as measured by DSC, because such methylpentene (co)polymer is superior in gas permeability.

In Examples described later, a methylpentene (co)polymer satisfying these physical properties is used.

Commercially available products can be readily obtained for use for all of the (a) styrene-butadiene hydrogenated block copolymer, (b) ethylene copolymer, and (c) methylpentene (co)polymer.

### (Ratio of resin components in formulation)

In the resin composition according to the present invention, the blending proportion of the hydrogenated block copolymer as component (a) is 30 to 40wt%. If the blending proportion is lower than 30wt%, the mixing properties of the composition is deteriorated. If the blending proportion is higher than 40wt%, the shaped sheet has lower strength against centrifugal force and lower breaking strength.

The blending proportion of the ethylene copolymer as component (b) is 35 to 65wt%. If the blending proportion is lower than 35wt%, the shaped sheet has lower strength against centrifugal force and lower breaking strength. If the blending proportion is higher than 65wt%, tackiness is generated on the shaped sheet.

The blending proportion of the methylpentene (co)polymer as component (c) is 5 to 25wt%, and preferably 5 to 15wt%. If the blending proportion is lower than 5wt%, the gas permeability, which is described later, is lowered. If the blending proportion is higher than 25wt%, the shaped sheet is hard and difficult to seal, or the transparency of the shaped sheet is lost, which complicate formation of a platelet storage container from the shaped sheet.

### (Preparation of composition)

The method for producing the resin composition with the above composition (hereinafter, occasionally referred to as elastomer composition) is not limited, and any method known as a conventional technique for blending a plurality of resin components (polymer components) may be used. To obtain the most homogeneous blend, for example, methods of melt-kneading with various kneading machines such as a mixing roll, a kneader, a Banbury mixer, and an extruder are preferred. In addition, it is preferable to dry-blend the materials to be blended by using a kneading machine such as a Henschel mixer and a tumbler in advance before melt-kneading, and then melt-knead the resulting mixture, because a homogeneous elastomer composition can be obtained.

### (Other components to be blended)

The resin composition according to the present invention may contain, in addition to the above essential main components, for example, a proper amount of a plasticizer, a silicone oil, an anti-blocking agent, a UV absorber, a lubricant, or a processing aid, in accordance with the application.

### (Shaping)

Applicable methods to form a container for medical use from the elastomer composition according to the present invention prepared as described above are, for example, a method in which the elastomer composition is first shaped into sheets by using inflation, a T-die, or calendering, and the resulting two sheets are stacked and heat-sealed (heat-sealed) to form a bag (sack), and a method in which the elastomer composition is blow-molded directly into the shape of a container by using injection molding or blow molding.

A platelet storage container obtained by shaping the resin composition according to the present invention into a sheet and then shaping the sheet into a bag in the above-described manner, or a storage container formed by blow-molding the resin composition according to the present invention can be connected to another container made of the same material or a container made of another material via a tube for use as satellite bags.

### (Thickness, L, of sheet)

The thickness of the sheet obtained by shaping the composition according to the present invention can be set in the range of 150 to 400 µm, and preferably in the range of 250 to 350 µm.

If the thickness, L, of the sheet is smaller than 150 µm, the physical properties of the sheet are insufficient, and the sheet is partially elongated or a pinhole is generated in centrifugation, and thus such a thickness is not preferred.

If the thickness, L, of the sheet is larger than 400 µm, shaping into a sheet is difficult, and even if a sheet is successfully obtained by shaping, the sheet has an excessively large thickness, which results in deteriorated flexibility and oxygen permeability lower than that required to enhance the platelet storability. Thus, such a thickness is not preferred.

### (Tensile properties)

With regard to tensile properties, the sheet obtained by shaping the composition according to the present invention has a 100% modulus of 2.7 to 5.0 MPa, a breaking strength of 20 MPa or higher, and an elongation of 550 to 750%. Accordingly, the sheet has sufficient tensile properties required for a sheet for a platelet storage container.

### (Oxygen permeability, Carbon dioxide permeability, Permeability ratio)

In the sheet obtained by shaping the composition according to the present invention, the oxygen permeability per unit thickness (PO₂) is 850 (ml·mm/m²·24h·atm) or higher, the carbon dioxide permeability per unit thickness (PCO₂) is 4,500 (ml·mm/m²·24h·atm) or higher, and the carbon dioxide/oxygen permeability ratio (CO₂ permeability/O₂ permeability) per unit thickness is 6.0 or lower.

Examination conducted by the present applicants has found that the oxygen permeability per unit thickness (PO₂) is enough if it is 850 (ml·mm/m²·24h·atm) or higher. This is because PO₂ of lower than 850 (ml·mm/m²·24h·atm) does not provide sufficiently high long-term platelet storability for high number of platelets such as 20 to 30 units, which the present invention targets, and thus is not preferred (as demonstrated in Examples described later, extremely high oxygen permeabilities per unit thickness (PO₂) of 1,000 or higher, and even of 1,100 or higher were actually obtained for sheets formed from the composition according to the present invention). Thus, a sheet having a preferred oxygen permeability satisfying PO₂ ≥ 850 (ml·mm/m²·24h·atm) can be achieved in the present invention.

The carbon dioxide permeability per unit thickness (PCO₂) can be suitably 4,500 (ml·mm/m²·24h·atm) or higher, and the carbon dioxide/oxygen permeability ratio (CO₂ permeability/O₂ permeability) per unit thickness can be suitably 6.0 or lower. If the permeability ratio exceeds this value, the permeability for carbon dioxide gas is unnecessarily high to the oxygen permeability. Hence, platelets cannot metabolize sufficiently in the early stage of platelet storage, and the amount of carbon dioxide gas generated from metabolism by platelets is smaller, and as a result the amount of carbon dioxide gas exiting the container exceeds the amount of carbon dioxide gas entering the container, leading to imbalance of the bicarbonate-based buffering action in the plasma, which results in increase of pH.

From such a viewpoint, it is preferable for prevention of increase of pH in the early stage of storage that the amount of carbon dioxide gas exiting the container and the amount of carbon dioxide gas generated from metabolism by platelets be equilibrated. To achieve this, it is suitable that the carbon dioxide/oxygen permeability ratio be 6.0 or lower, and preferably 5.5 or lower. As demonstrated in Examples described later, the permeability ratio (CO₂ permeability/O₂ permeability) of the sheet according to the present invention is 5.5 or lower for all cases.

In summary, the setting of the thickness, PO₂, and PCO₂ of the sheet obtained by shaping the composition according to the present invention within the above ranges provides the best effect in terms of platelet storability, gas permeability, and the processability of the sheet.

As described hereinbefore, the resin composition according to the present invention, which contains (a), (b), and (c), can be shaped not only into a sheet, but also into a tube, for example, by using an extruder. In addition, the resin composition according to the present invention can be subjected to injection molding and blow molding, and can be shaped into the shape of what is called bottle. Thereby, a platelet storage container as a bottle, not only a platelet storage container as a bag, can be produced.

### Examples

Now, the present invention will be described in more detail with reference to Examples. However, these Examples are each an example of embodiments of the present invention, and the present invention is never limited to these Examples.

Components of the (a) styrene-butadiene block copolymer, the (b) ethylene copolymer, and the (c) methylpentene (co)polymer were blended in accordance with compositions shown in Table 1 (Examples 1 to 9) to prepare resin compositions. The resin compositions were shaped into sheets having different thicknesses in the range of 250 to 350 µm. Tests described below ((1) test on gas permeability, (2) test on physical properties, (3) test on seal strength, (4) evaluation test on platelet adhesion) were conducted for these sheets, and the results are shown in Tables 1 to 4. The (c) methylpentene (co)polymer used had MFR: 15 g/10 min and p: 850 g/cm³.

As shown in Table 1, a resin composition containing polypropylene instead of the (c) methylpentene (co)polymer was shaped, as Comparative Example 1, in the same manner as in Examples 1 to 9, and the resultant was subjected to the tests. The results are shown together in Tables 1 to 4.

### (1) Test on gas permeability (Table 2)

The gas permeability per unit thickness (ml·mm/m² ·24h·atm), P, of each of the sheets produced in the above-described manner was measured in accordance with JIS K7126-1 with the measuring apparatus GTR-31A (manufactured by GTR TEC Corporation) at a temperature of 23°C. The results are shown in Table 2. It was confirmed from the results shown in Table 2 that the oxygen permeability and carbon dioxide gas permeability in any of Examples 1 to 9 of the present invention were much better compared to those in Comparative Example 1. In addition, the carbon dioxide/oxygen permeability ratio (CO₂ permeability/O₂ permeability) was found to be 6.0 or lower, and even 5.5 or lower, in a stable period. These results are examined in detail in the section of Discussion described later, where it will be demonstrated that the oxygen permeability (together with the carbon dioxide gas permeability) is far higher than that in PTL 1 previously disclosed by the present applicants.

### (2) Test on physical properties (Table 1)

The 100% modulus, tensile strength at break, and tensile elongation at break of each of the sheets produced in the above-described manner were measured in accordance with JIS K6301 and JIS K7127. The results are shown in Table 1. It was confirmed from the results shown in Table 1 that the values in Examples 1 to 9 of the present invention and those in Comparative Example were comparable, and large tensile stress at break was exhibited, which indicates superiority in tensile strength, in both Examples and Comparative Example. Specifically, the sheets in Examples of the present invention were all found to have a 100% modulus in the preferred range of 2.7 to 5 MPa, tensile elongation at break in the preferred range of 550 to 750%, and tensile strength at break in the preferred range of 20 MPa or higher.

### (3) Test on seal strength (Table 3)

The sheets in Examples 1 to 5 and Comparative Example were prepared as representative examples, and a pair of sheets were heat-sealed for each of Examples and Comparative Example, and subjected to a tensile test to examine the seal strength. The results are shown in Table 3. It was confirmed from the results shown in Table 3 that the seal strength was high and the tensile strength at break was superior for all of Examples 1 to 5 of the present invention and Comparative Example. In particular, the seal strength is maximized at around 220°C. However, it should be noted that a high seal temperature is needed to give such a value of seal strength in Examples 4 and 5.

### (4) Evaluation test on platelet adhesion (Table 4)

An evaluation test on platelet adhesion was conducted for Examples 1 to 5 and Comparative Example as representative examples.

The test on platelet adhesion was conducted in the following manner: a 5 mm × 15 mm sheet obtained by shaping as described above was soaked in 2 mL of a test solution with the platelet concentration adjusted to 3.0 ± 0.2 × 10⁵ cells/µL, and incubated at 37°C for 1 h; the sheet was then quickly rinsed with ultrapure water, and thereafter 5 mL of 2.5wt% glutaraldehyde aqueous solution was added thereto and the resultant was left to stand for 24 hours; after air-drying, SEM observation was performed to count platelets adhering to the surface.

Table 4 shows the results on the number of platelets adhering to both sides of each 5 mm × 15 mm sheet as a test piece. It was found from the results shown in Table 4 that the number of platelets adhering to each test piece was 0.07% or less of the number of platelets in the test solution for soaking. Thus, it was confirmed that the number of platelets was small for all of Examples 1 to 5 of the present invention and Comparative Example 1 and the sheets in Examples 1 to 5 and Comparative Example 1 are superior in resistance to platelet adhesion.

The above results demonstrate that the sheet for a platelet storage container (each of Examples 1 to 9) obtained by shaping the composition according to the present invention is superior in strength (resistance to mechanical force), molding and/or forming properties, and resistance to platelet adhesion, and, in particular, significantly superior in oxygen permeability, and hence is expected to enable further continuous and stable long-term storage of high number of platelets.

### (Formulation table and results of test on physical properties)

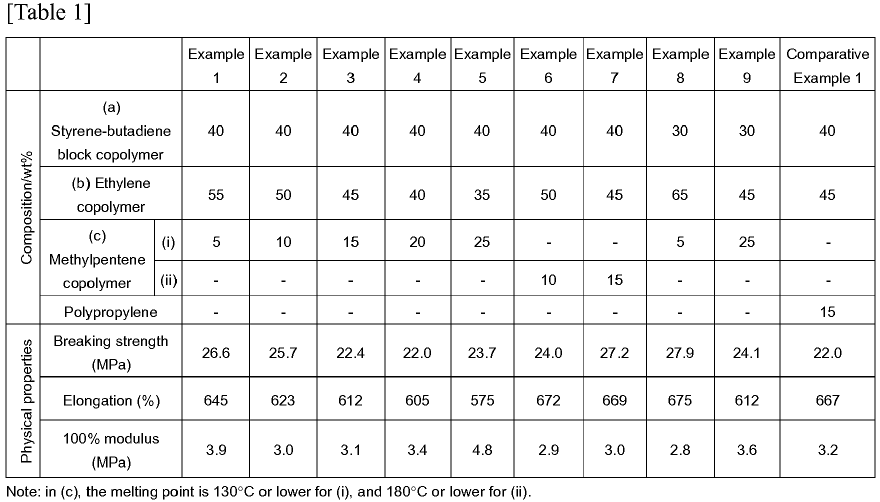

### (Results of measurement of gas permeability)

**[Table 2]**

| | (ml·mm/m²·day·atm) | | CO₂/O₂ |
|---|---|---|---|
| | O₂ permeability | CO₂ permeability | |
| | AVE | AVE | |
| Example 1 | 947 | 4897 | 5.2 |
| Example 2 | 1055 | 4849 | 4.6 |
| Example 3 | 1135 | 5066 | 4.5 |
| Example 4 | 1144 | 5504 | 4.8 |
| Example 5 | 1188 | 5561 | 4.7 |
| Example 6 | 970 | 5034 | 5.2 |
| Example 7 | 1056 | 5098 | 4.8 |
| Example 8 | 865 | 4382 | 5.1 |
| Example 9 | 1094 | 5175 | 4.7 |
| Comparative Example 1 | 749 | 3395 | 4.5 |

### (Results of measurement of seal strength)

**[Table 3]**

| Seal strength [N/10 mm] | | | | | | | | |
|---|---|---|---|---|---|---|---|---|
| MD | 180°C | 190°C | 200°C | 210°C | 220°C | 230°C | 240°C | 250°C |
| Example 1 | 26.34 | 28.11 | 29.23 | 30.18 | 32.18 | 30.27 | 29.84 | 33.75 |
| Example 2 | 19.20 | 23.38 | 29.03 | 30.08 | 30.95 | 30.27 | 34.24 | 33.67 |
| Example 3 | 16.06 | 19.49 | 24.51 | 28.72 | 32.63 | 31.28 | 31.59 | 32.22 |
| Example 4 | 10.78 | 12.53 | 16.00 | 24.37 | 23.93 | 30.98 | 33.25 | 33.50 |
| Example 5 | 8.16 | 11.51 | 13.88 | 19.79 | 26.76 | 28.79 | 33.49 | 31.20 |
| Comparative Example 1 | 26.44 | 28.43 | 32.22 | 40.08 | 36.79 | 37.80 | 38.15 | 38.12 |

### (Results of measurement of platelet adhesion)

**[Table 4]**

| | Example 1 | Example 2 | Example 3 | Example 4 | Example 5 | Comparative Example 1 |
|---|---|---|---|---|---|---|
| N=1 | 283554 | 425331 | 170132 | 113422 | 241021 | 311909 |
| (cells) | | | | | | |
| N=2 | 198488 | 311909 | 326087 | 241021 | 113422 | 368620 |
| (cells) | | | | | | |
| N=3 | 170132 | 425331 | 311909 | 85066 | 297732 | 226843 |
| (cells) | | | | | | |
| Average | 217391 | 387524 | 269376 | 146503 | 217391 | 302457 |
| (cells) | | | | | | |
| Adhesion rate | 0.036 | 0.065 | 0.045 | 0.024 | 0.036 | 0.050 |
| (%) | | | | | | |

| | | | | | | |
|---|---|---|---|---|---|---|
| Note: per 150 mm² (the surface area of both surfaces of a test piece) | | | | | | |

### (Discussion)

The O₂ permeability (together with the CO₂ permeability) will be specifically examined from the above results in Examples.

It is particularly noticeable in Table 2 that the O₂ permeability per unit thickness (PO₂) [(ml·mm/m²·24h·atm)] in each of Examples 1 to 9 is at least 850 or higher, preferably 1,000 or higher, and more preferably 1,100 or higher, which is a high value not found in conventional techniques. The CO₂ permeability (PCO₂) is at least 4,300 or higher, preferably 4,800 or higher, and more preferably 5,000 or higher, which is also an extremely high value.

In PTL 1 previously disclosed by the present applicants, the O₂ permeability (PO₂) and CO₂ permeability (PCO₂) in Example 6, as a representative example, are PO₂ = 703, PCO₂ = 3,194 (PTL 1, Table 1), and those in Example 10 are PO₂ = 545, PCO₂ = 2,454 (the same literature, Table 7), and these values are far lower than the maximum permeabilities in Examples herein, PO₂ = 1,100, PCO₂ = 5,000.

The pH change when a platelet concentrate was stored in a container having such values of permeability (PO₂ and PCO₂) for 120 hours was (pH: from 7.09 to 7.24) in storage of 10 units and (pH: from 7.15 to 7.14) in storage of 20 units for Example 6 (the same literature, Table 5), and (pH: from 6.97 to 7.16) in storage of 10 units and (pH: from 7.03 to 6.91) in storage of 20 units for Example 10 (the same literature, Table 10).

On the basis of these results, the present invention provides a far higher oxygen permeability (PO₂) than PTL 1 as a prior art by the present applicants, and the PCO₂/PO₂ balance is stably kept within the preferred range, and hence the present invention is reasonably expected to be effective for storage of 20 units, needless to say, and even for storage of 30 units.

Here, a further examination is made in this regard. Platelets operate the anaerobic glycolytic pathway to produce lactic acid when the O₂ level (dissolved O₂ concentration) is low in the system. When the lactic acid concentration increases, the pH value decreases and the platelet functions are lowered. Therefore, formation of a platelet storage container with a sheet having high O₂ permeability is of significant importance to prevent decrease of pH. The platelet storage container according to the present invention has high O₂ permeability which is not found in conventional techniques (in addition, the CO₂/O₂ balance is kept within the preferred range), and hence is expected to be usable suitably as a platelet storage container for storage of high number of platelets.

### Industrial Applicability

When the composition for a platelet storage container according to the present invention is shaped, for example, into a sheet for use, the shaped sheet is superior in strength (resistance to mechanical force), molding and/or forming properties, and resistance to platelet adhesion, and, in particular, significantly superior in oxygen permeability, and the carbon dioxide/oxygen permeability ratio (CO₂/O₂) is kept well-balanced within a preferred range in spite of the high oxygen permeability. Accordingly, the shaped sheet enables further continuous and stable long-term storage of high number of platelets, and the applicability in the field of medical industry is extremely high.

## Claims

1. A composition for a platelet storage container, the composition comprising:
(a) 30 to 40wt% of a block copolymer prepared by hydrogenation of a block copolymer comprising a polymer block A comprising at least two vinyl aromatic compounds and a polymer block B comprising at least one conjugated diene;
(b) 35 to 65wt% of polyethylene obtained by copolymerizing ethylene and a C₃₋₁₂ α-olefin with a metallocene catalyst, the polyethylene having a density of 0.860 to 0.900 g/cm³ and a maximum melting peak temperature (Tp) of 90°C or lower as measured by differential scanning calorimetry (DSC); and
(c) 5 to 25wt% of poly(methylpentene) or a copolymer comprising methylpentene with ethylene or an α-olefin,
wherein the component (c) has a Melt Flow Rate (MFR) of 3 to 20 g/10 min, a density of 800 to 900 g/cm³, and a melting point (Tm) of 180°C or lower as measured by the DSC.

2. A sheet for a platelet storage container, the sheet obtained by shaping the composition according to claim 1, wherein:
a thickness of the sheet is 150 to 400 µm, and
an oxygen permeability per unit thickness of the sheet is 850 ml·mm/m²·day·atm or higher, a carbon dioxide permeability per unit thickness of the sheet is 4,500 ml·mm/m²·day·atm or higher, and a carbon dioxide/oxygen permeability ratio (CO₂ permeability/O₂ permeability) per unit thickness of the sheet is 6.0 or lower.

3. The sheet for a platelet storage container according to claim 2, wherein:
a 100% modulus of the sheet is 2.7 to 5 MPa,
a breaking strength of the sheet is 20 MPa or higher, and
an elongation of the sheet is 550 to 750%.

4. A platelet storage container obtained by forming the sheet for a platelet storage container according to claim 2 or 3 into a bag.

5. A platelet storage container obtained by shaping the composition according to claim 1 into a tube and then shaping the tube into a bottle, or obtained by shaping the composition into a bottle.
